(19) Europäisches Patentamt
European Patent Office
Office européen des brevets

(11) **EP 3 753 482 A1**

(12) **EUROPEAN PATENT APPLICATION**

(43) Date of publication:
**23.12.2020 Bulletin 2020/52**

(51) Int Cl.:
**A61B 5/0205** (2006.01)     **A61B 5/024** (2006.01)
**A61B 5/08** (2006.01)     **A61B 5/11** (2006.01)
**A61B 5/00** (2006.01)     A61B 5/04 (2006.01)

(21) Application number: **20175446.2**

(22) Date of filing: **19.05.2020**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **21.05.2019 FI 20195420**

(71) Applicant: **Firstbeat Analytics OY**
**40100 Jyväskylä (FI)**

(72) Inventors:
• **SAALASTI, Sami**
**40100 JYVÄSKYLÄ (FI)**
• **RUHANEN, Tuukka**
**40100 JYVÄSKYLÄ (FI)**

(74) Representative: **Kespat Oy**
**Vasarakatu 1**
**40320 Jyväskylä (FI)**

(54) **METHOD AND APPARATUS FOR DETECTING A SLEEP STATE**

(57)     The object of the invention is a method and device for detecting a sleep state by means of heart-rate and movement data. By means of neural network software, a sleep state is detected using as input data movement data (move_count) and data derived from heart-rate data and/or data derived from inter-beat interval data such as (MAD) and respiration data (RESP). In the method, at least a portion of the variables (HRD, MHR, MAD, Resp, GRD) derived from the heart-rate data/inter-beat interval data are further modified by means of one, most preferably 2-4, artificial average functions and the cumulative sleep time is one input datum.

Fig. 1

**EP 3 753 482 A1**

**Description**

The object of the invention is a method and apparatus for detecting a sleep state by means of heart-rate and movement data. In the method, a correlation software is used for detecting the sleep state. The input data are the data derived from heart-rate and inter-beat interval data as well as movement data. The invention is also directed to an auxiliary logic with which the detection of a sleep state with the basic method can be clearly improved.

Sleep states have been detected for some time now in the aforementioned manner. The problem with simple calculations is their unreliability. Complicated calculations, which can even have hundreds of variables, require significant calculation resources. Moreover, the calculation is frequently completed after a significant delay.

The article *"Estimation of sleep stages in a healthy adult population from optical plethysmography and accelerometer signals"* (Physiol Meas. 2017 Oct 31;38(11):1968-1979. doi: 10.1088/1361-6579/aa9047*; [FITBIT],* Beattie Z. et al; Fitbit Research, San Francisco, CA 94105, Unites States of America*)* describes possibilities of wrist-worn devices in detecting sleep.

Beattie et. al. "Estimation of sleep stages in a healthy adult population from optical plethysmography and accelerometer signals" (2017. [FITBIT]) reported the utilization of 54 separate input variables in its sleep stage classification.

Neural network calculations have been used for some time for the modelling of complex, generally non-linear phenomena. *Per se,* the final result generated by the neural network can be a markedly simple function with respect to the calculation process required for its creation and considering the large quantity of empirical data. Modelling requires laborious calculations and a considerable amount of empirical material for the creation of a model. The problem of known neural networks is, however, that there is typically a lag of 40min-60min in sleep states that they output, as neural networks need data about both past as well as future sleep in order to be able to identify the sleep state at the current moment in time correctly.

Neural network calculations can be realized, for instance, with the Matlab software Neural Network Toolbox. In particular software of the feedforward neural network variety is best suited to this field. The result is a neural network which can be adapted for portable devices such as wrist-worn and PDA devices as well as smart phones. By means of the neural network, the created function is markedly simple in comparison with the resources required for its formulation.

The purpose of the invention is to create a neural network calculation which is simpler, is not delayed and by means of which, in addition to the elimination of any delay, a significant improvement in accuracy is achieved in comparison with known real-time calculations. The essence of the object of the invention is achieved with the features indicated in claim 1.

In the simplest embodiment of the method, a correlation function is used in order to detect a sleep state for which the input data is movement data (move_count), data derived from heart-rate data, such as MHR, HRD, usually in terms of beats per minute (bpm), data derived from inter-beat interval data, such as MAD, usually in terms of milliseconds (ms), and cumulative sleep time. It is most advantageous to also consider additional data derived from heart-rate data, such as GRD, and additional data derived from inter-beat interval data and respiration data (RESP) at least at night, as the PPG is able to measure this data in conditions without movement. Naturally, it is also possible to obtain all major data in better quality during the day with an ECG device.

In one embodiment of the method, as few as 7 input variables for the correlation function are used. In such a case, the input data can include move count, HRD, MHR, MAD, GRD, respiration, and cumulative sleep time. The input variables can be further modified by 0 - 4 artificial average functions having different weightings. When the average functions are used, the accuracy of the sleep state detection can be increased notably. The total number of input variables for the correlation function can be 7 - 28, preferably 17 - 23. Preferably the input variables include at least one variable depicting low frequency (LF, 0.04 - 0.15 Hz) physiological features, such as HRD, and at least one variable depicting high frequency (HF, 0.15 - 0.4 Hz) physiological features, such as MAD.

Various classifier techniques, such as neural networks, nearest neighbor classifier, random forest classifier, support vector machine, decision tree, and linear discriminant classifiers, can be used to obtain the correlation function. According to one advantageous embodiment, the correlation function is a neural network, e.g. a feed-forward neural network. A neural network can "learn" non-linear correlations of input variables from empirical data and produce a simple correlation function with respect to the calculation process required for its creation and considering the large quantity of empirical data. A feed-forward neural network is the simplest type of artificial neural network devised and can produce an accurate correlation function to be used with limited memory and calculation power resources.

The correlation function can be divided into subfunctions. In one embodiment of the method, the correlation function includes at least two subfunctions where the first subfunction detects only sleep states "sleep" & "non-sleep", and where the rest of the subfunctions handle other sleep states. Also, there can be another subfunction detecting awake state from other sleep states. This simplifies the calculation and yields more accurate results for the sleep state detection.

According to one advantageous embodiment, in addition to a real-time calculation, a dynamic calculation with a delay is used, which improves the calculation of the entire time with a short delay. Corrective calculations realized by means of an auxiliary logic can also be applied in other contexts than the context of the main method described in the foregoing.

The detection of a sleep state from movement-sensor, heart-rate data and inter-beat-interval data can be implemented efficiently with a real-time neural network method without a delay. Movement and heart rate, as well as variables produced by heart-rate variability, enter into the neural model as input data while the output datum is the current sleep state in real time SLEEP_STAGE; 0 (no sleep), 2-5 (DEEP/LIGHT/REM/AWAKE). The accuracy of the method can be increased by storing personal background parameters relating to a minimum heart rate, as well as heart-rate variability at night. A typical input variable can be e.g. a current heart rate that is reduced by a minimum heart rate (MHR), i.e. the distance of the heart rate from its minimum rate, and the moving average (MA) calculated from the same $f\_ma(t)=(c*f\_ma(t-1)+MHR(t))/(c+1)$, where coefficient c is typically 20-80 with a sample window of 5s depending on the degree of smoothness desired. Moreover, a part of the input variables can be calculated with discrete moving average windows, which yields additional accuracy for the method. The moving averages calculated with discrete moving average windows for individual variables correct the characteristic feature of normal neural networks, which are linked with the requirement of obtaining data for past and "future" points in time. The distinctive feature of this invention is that, from the point of view of the user, a disadvantageous delay is not necessary, because the neural network receives the "future data" from moving averages calculated with different window lengths.

A distribution of the heart-rate variability variables using an average night-time heart-rate variability also improves accuracy. The implementation can include two different neural models for the detection, wherein the first (the less accurate) model is used when the night-time background parameters have not yet been calculated, while the second method is used when the background parameters have been calculated.

The real-time detection of a sleep state is associated with inaccuracies which can be corrected with a delay by means of a so-called dynamic delay. Inaccuracies are frequently related to random events associated with the current heart rate, heart-rate variability or movement, during which the sleep state is momentarily altered. The sleep state is nevertheless classified in a continuous fashion and interruptions are not desirable (e.g. momentary LIGHT sleep within REM sleep). Furthermore, a classification of momentary episodes of waking up during sleep (AWAKE) is desired as opposed to the method indicating that sleep has ended (SLEEP_STAGE=0). During the day, it is typically possible to identify momentary sleep states that one wishes to remove from the analysis.

The situations described above can be improved in a delayed manner by means of the following methods:

- By storing the last 30 sleep states e.g. with a resolution of a minute in a loop buffer (30min buffer, 3-8bit read resolution). It is possible to calculate windowed averages for the states from the buffers so that momentary state variations can be eliminated by means of the average. In this case, the dynamic delay is half the length of the window if the average state and the momentary state differ so as to require a correction. Said averages can be replaced by physiological rules.
- By storing significant sleep state changes (sleep begins, sleep ends, sleep continues) in time indexes, it is possible to draw logical conclusions from the same in spite of an AWAKE state that constitutes a point of sleep interruption.
- By means of rules: e.g. if a sleep state (<15min) is detected in an even slightly cumulative manner and a "waking" becomes evident based on movement and heart rate, the detected sleep is erased. A simple logic is required in the evening so that e.g. lying on the couch and/or reading in bed is readily detected as momentary sleep. In practice, sleep can be registered as beginning when movement subsides. This significantly improves the situations described above.
- Rule: momentary AWAKE states, however, are not corrected within sleep. AWAKE states constitute a fragmentation that occurs naturally within sleep.

Typically, a reasonable time resolution is a5pprox. 30s-1min when detecting sleep states. During implementation, changes in sleep states are identified in a subsequent calculation and the identified change (new state, uint8 stage), time of change (uint16 t) and length (uint16 len) are transmitted to an interface which limits a correction outside a normal detection frequency. If the detection yields a new real-time sleep state in an even interval, e.g. every 30s, the corrections can be allocated within this time segment. A detection occurs by simply defining the length as deviating from zero (len>0). For the purposes of a graphic representation, it is possible to produce a final sleep-state graph from the time series stage,t,len with a simple function (Matlab®):

```
function y=sleep_reconstruct(stage,t,len),

y=stage; lst=y(1);

for i=1:length(stage),

    if len(i)>0,

        for j=i-t(i)-len(i)+1:i-t(i),

            if stage(i)<1 | y(j)~=5,

                y(j)=stage(i);

            end;

        end;

        y(i)=lst;

    else

        y(i)=stage(i);

        lst=y(i);

    end;

end;
```

It is significant that sleep reconstruct() can be said to occur in real time when e.g. an image to be displayed in real time is updated and prior moments in time can then change in the detection. However, the further away the points of measurement are from the current point in time chronologically, the more likely it is that they will no longer change.

In practice, a time window of a chosen length, e.g. a five-minute time window, is applied to an incoming signal, whereupon an auxiliary logic corrects the incoming signal by means of predetermined rules. First, the episodes making up the bulk of one sleep state are sought in the incoming signal.

According to one main rule, it is possible to change the entirety of a given data segment into one given sleep state if the segment in question constitutes e.g. at least 80% of the sleep state in question. The entire episode can thus be changed into a given same sleep state according to a more precise rule if the bulk of the points in time in the segment in question belong to the same sleep state.

One advantageous embodiment takes advantage of the utilization of the night-time averages of the heart-rate variability variables and, moreover, the possibility according to the invention of switching between models: In order to correct future samples, it turns out to be a useful strategy to implement a scaling (distribution) of the heart-rate variability variables with the night-time averages. Still in practice, this can be realized in such a way that, when measuring the first night, a less accurate neural model is used for the detection of a sleep state and an average of the heart-rate variability variables is stored in real time. For example, the first 4h of the night are a sufficient sample for the variables to stabilize. It is subsequently possible to switch to using a neural model which utilizes these averages. The method is 6approx. 2% less accurate during the first night because the main part of the information regarding variability is utilized in distinguishing between REM sleep and LIGHT sleep. REM sleep is typically more common at the end of the night while deep sleep (DEEP) is more common at the beginning of the night. Precisely the variability averages with their errors do not benefit the detection of deep sleep.

The invention is described in the following with reference to the attached drawings in which certain applications of the invention and details of the calculation are shown.

Figure 1 shows an advantageous neural network model for defining a sleep state
Figure 2 shows a typical hardware configuration of a wrist-worn device with its interfaces
Figures 3a - 3g show input values of the neural network during measurement, of which

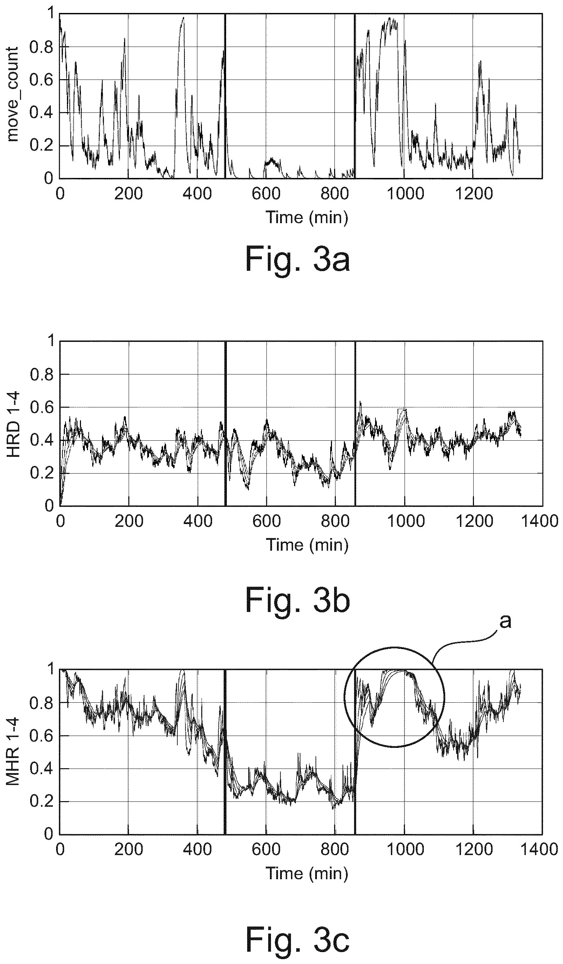

Figure 3a shows a move_count variable expressing a state of movement
Figure 3b shows an HRD variable and a variable modified from the former with three delays
Figure 3c shows an MHR variable and a variable modified from the former with three delays

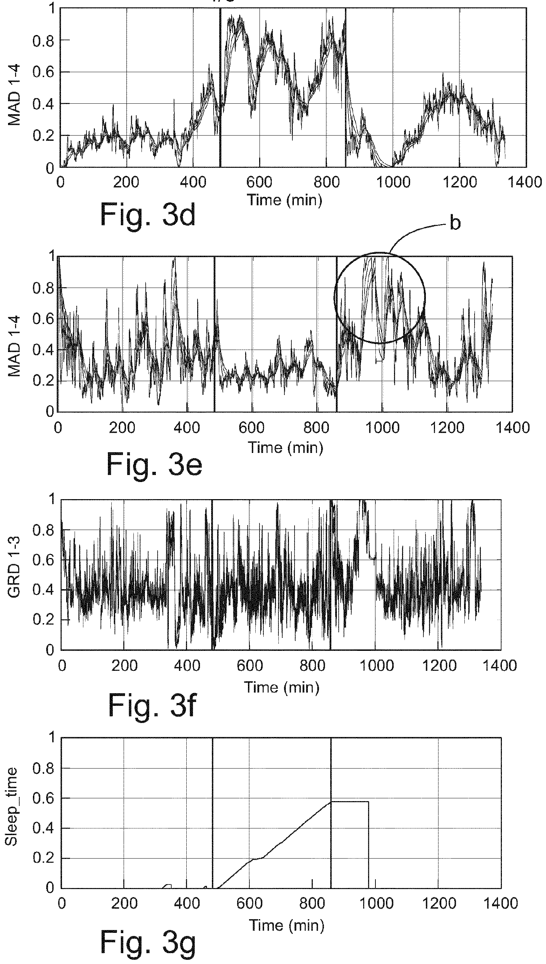

Figure 3d shows an MAD variable and a variable modified from the former with three delays
Figure 3e shows a RESP variable and a variable modified from the former with three delays
Figure 3f shows an GRD variable and a variable modified from the former with three delays
Figure 3g shows a sleep_time variable representing the cumulated sleep state

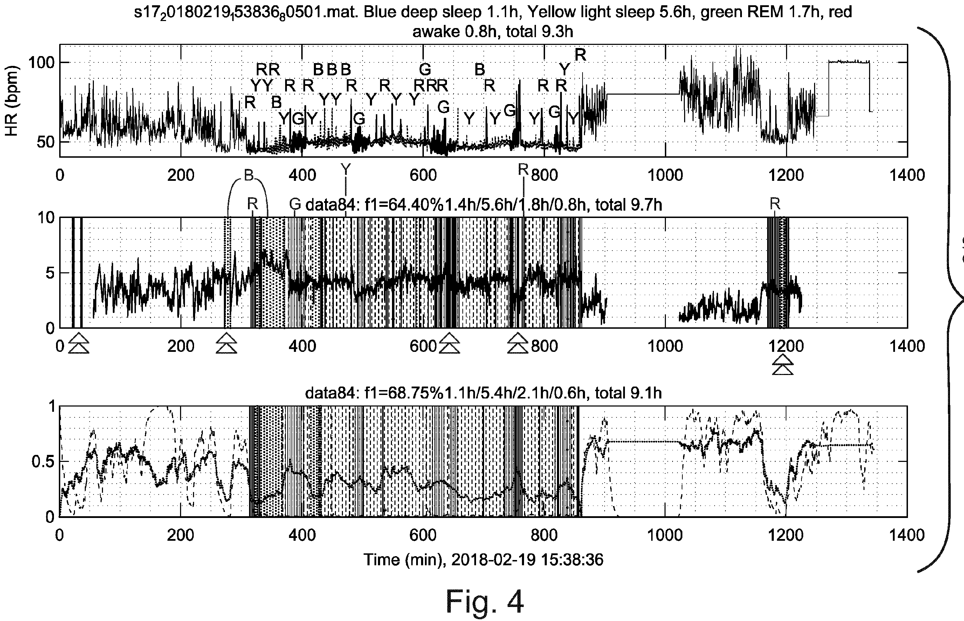

Figure 4 shows the progression of a calculation from measurement signals

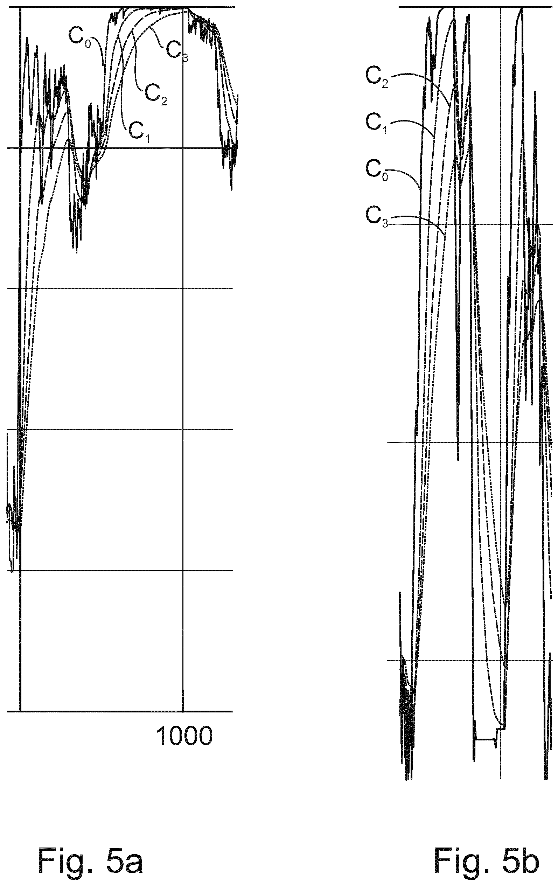

Figure 5a shows plots of the delay-calculated signals from Figures 3c (section a)
Figure 5b shows plots of the delay-calculated signals from Figures 3e (section b)

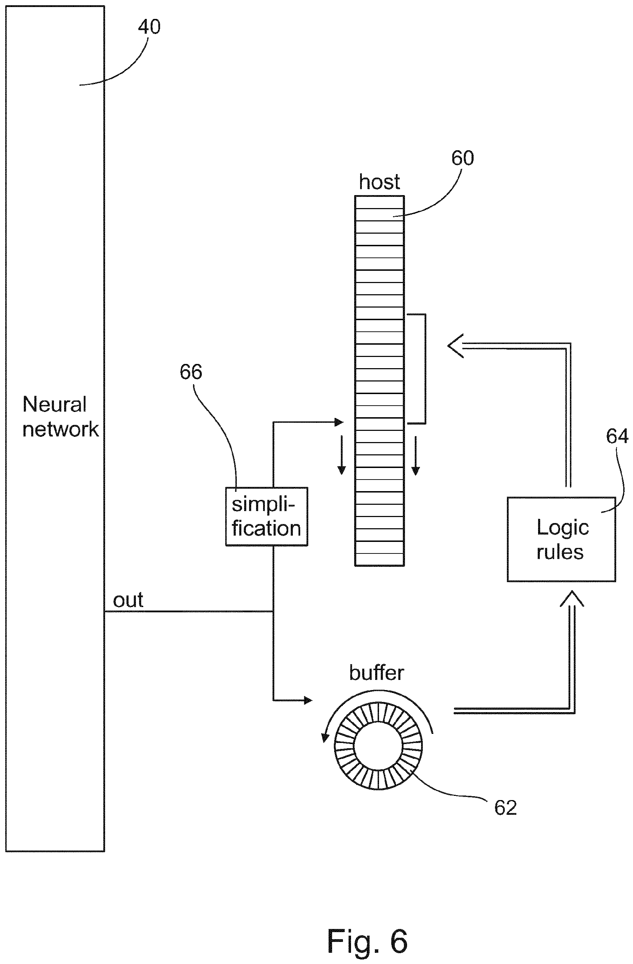

Figure 6 shows the processing of the output of the neural network

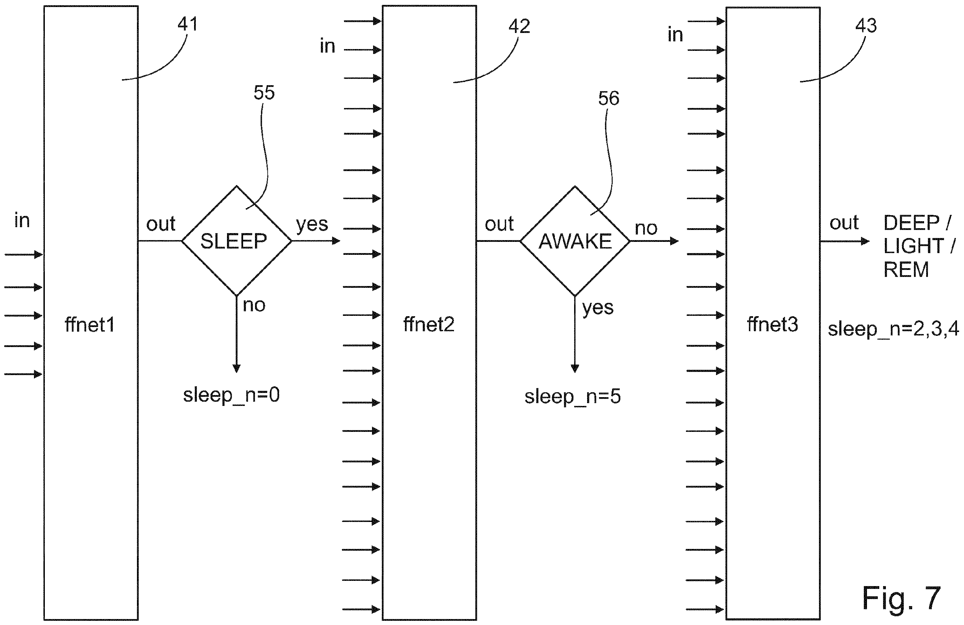

Figure 7 shows a block diagram describing the sleep state detection in thee subfunctions

The above abbreviations are HRD = "Heart Rate Deviation", MAD= "Mean Absolute Difference between successive RR-intervals"", MHR=" Heart rate difference (from Minimum HR)", RESP = Respiration (respiratory rate), PPG="Photoplethysmogram", GRD="Gradient of the heart rate difference".

Figure 4 shows the progression of a calculation from measurement signals (top bar) to a real-time calculation (middle bar) and a final result obtained by means of a dynamic-delay calculation (bottom bar). Figures 5a and 5b illustratively show plots of the delay-calculated signals from Figures 3c (section a) and 3e (section b). These are a pivotal feature of the basic invention. Together with the chosen group of variables, the neural network calculation produces a markedly accurate detection of a sleep state with a relatively small quantity of input data.

The fundamental idea of neural networks is based on natural neural networks. However, generally speaking, the objective these days is not a precise simulation of natural neural networks, but rather the development of neural network techniques is based more on, for instance, statistical science and signal-processing theory.

There are numerous alternatives with respect to the software that can be chosen for neural network applications. The above-mentioned Matlab version is well suited to this purpose.

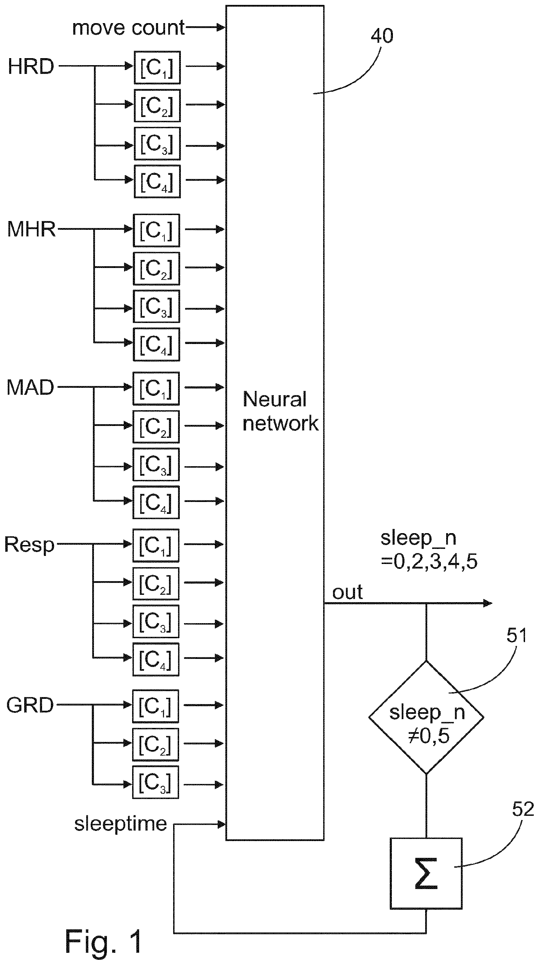

The neural network 40 application of Figure 1 is quite simple, as there is a very limited number of input data, namely 21. There is only one output datum, i.e. the sleep state, which is stored to a result register. Different wrist-worn device manufacturers implement neural network 40 software configured for these devices.

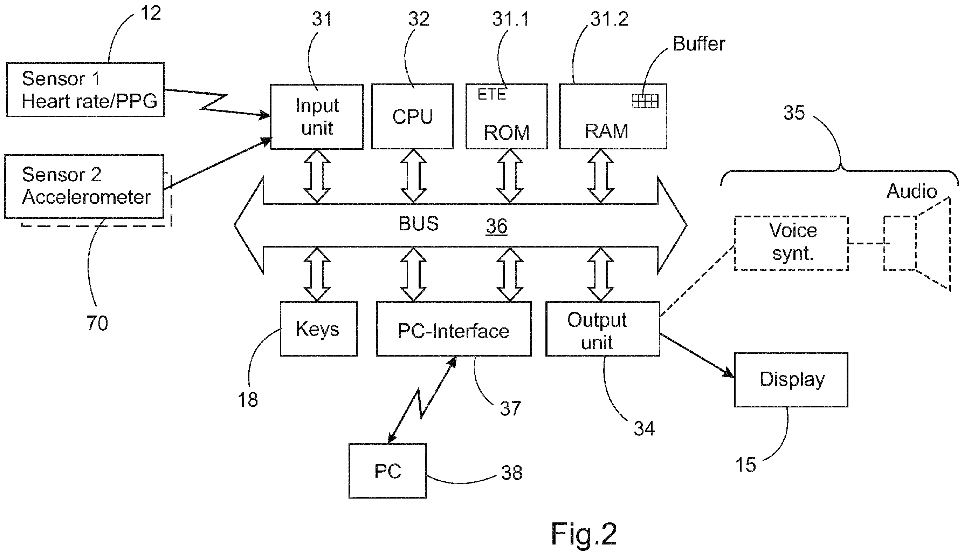

In accordance with the invention, the method can be realized in particular with a very conventional wrist-worn device, the hardware of which is depicted in Figure 2. The apparatus, here a wrist-worn device, includes the required measurement sensors 12, 70 (heart-rate and acceleration sensors), a bus channel 36, keys 18 and a unit 31 for inputting data, a central processing unit 32, ROM memory 31.2 and RAM memory 31.2, including a buffer (loop buffer memory), an output unit 34, a display 15 as well as potential voice synthesizers and speakers 35. The device generally has an interface 37 for interfacing with a PC 38 or for connecting directly to the internet. In the following, the memory sizes required by the invention are explained. The buffer memory required by the auxiliary logic constitutes a relatively small resource.

From the sleep-state classification, it is typically desirable to store and share with the user *inter alia* the average ratios and/or times of the sleep stages, the time of falling asleep, as well as the total time slept. The dynamic delay described above can change said variables generated in real time. Whenever the sleep state is changed after the fact, it is possible to update the internal counters in real time (e.g. the cumulative totals of the sleep stages). Moreover, the final results are typically not called up during sleep, but after sleep has ended so that the final results are generally ready or will no longer be subject to significant changes. Potential inaccuracies resulting from the dynamic delay are small compared to typical errors caused by machine learning when classifying sleep states. The empirically (n=110 expert-classified sleep data sets) described method is in real time 93% of the time, while the average correction delay is 8.6 min the remaining 7% of the time.

Reference is made to Figure 4 in the following. In this illustrative figure, the expert-classified sleep states are in the top graph, the real-time sleep states provided by the neural model are in the middle and the sleep states provided with a delay are in the bottom graph.
When comparing the bottom and middle graph, one observes:

- The momentary incorrect detections occurring at the points 24min, 278min, 1188min have been erased.
- At the point 638min, the fragmentation of REM sleep has been smoothed over, thus improving accuracy.
- The sleep interruption at the point 767min has been corrected to AWAKE.
- By means of the dynamic delay, the average accuracy of sleep detection improved from 64.40% => 68.75% ($F_1$ score).

In addition to the improved accuracy, a significant benefit of the method resides in the subsequent graphic display of the sleep states to the user. It is possible to implement the method efficiently in a wrist-worn device in which the calculated detection of a sleep state in real time also renders possible the displaying of the results of the sleep analysis (time slept, sleep feedback, time of falling asleep, sleep state distributions) immediately after waking up. By means of an efficient coding, the transmission and display of the results e.g. on a mobile device with a larger monitor is simple and the results are identical with the results of the wrist-worn device.

To our knowledge, a method of the type described that enables an operation in real time in a wrist-worn device does not exist on the market, but rather the analyses occur after the fact e.g. with a mobile device. Moreover, some of the methods (such as Polar) generate results about 1h after sleep has ended. The described method enables the generation of feedback regarding sleep times on the wrist-worn device immediately after sleep has ended. The feedback information at this stage can also be a verbal text produced by an expert system and a higher-level classification further including guidance for ameliorating potential observed sleep irregularities (short sleep, long time falling asleep, numerous sleep interruptions).

Empirical testing

For the production of the generated error values and figures in this illustration, a sleep data population (different test persons) of n=110 was used in which actual sleep states were detected in a sleep laboratory using more extensive sensor data, such as an electroencephalogram. In addition, the modelling database includes n=150 expert classified sleep start/end-times, n=781 user estimated sleep start/end-times. The intention is to detect sleep states with a heart-rate device as well as possible solely on the basis of sensor data pertaining to heart rate and heart-rate acceleration.

Expanding the input variable time window into the past and future in a real-time system for sleep state detection

Based on empirical and physiological research, sleep state detection benefits from entering temporal data relating to the input properties into the neural model. The so-called $F_1$ score, also known as the Sørensen-Dice coefficient or Dice similarity coefficient (DSC), accuracy significantly improves if, in addition to samples calculated in real time from input vectors, samples relating to the past (e.g. -40min -20min from the current point in time) as well as the future (+20min +40min from the current point in time) are entered into the neural model. Typically, the $F_1$ score improves for both operations by an average of 10approx. 10%. The detection of REM sleep particularly requires temporal information in order for the neural model to be able to relate the current heart rate variability and heart rate to the average changing rate over time.

Although the described sample window improves the accuracy of the system, it produces undesirable attributes in a system that works in real time in a small device: an input variable sample window over time requires a significant use of memory (altogether as much as an 80min buffer for each input variable) while the future samples create a delay (40min in the examples) for the real-time system.

The aforementioned weakness can be bypassed by outputting input variables that yield corresponding information for the neural model. The samples relating to the past can be corrected by calculating the input variables with different moving average (MA) windows (increasing the constant c). In the illustrative figure, figure 3c, four different averages ($c_1$=20, $c_2$=70, $c_3$=140, $c_4$=210) have been calculated from the input variable MHR. The input variables have been scaled between 0-1. It is evident in the figure that the addition of the moving average smooths out the variable in addition to the temporal displacement. The operation thus does not naturally correspond to the collection of the samples from the original variables at different points in time. Via empiricism, however, it can be observed that the solution is able to correct the sample window and provide the neural model with sufficient temporal information regarding the past. It is also possible for the neural network to utilize input variables generated in this manner *inter alia* in order to render the temporal resolution more precise and to distinguish between input variable gradients.

Figure 5a shows the inset a from Figure 3c. In this figure, the lag of the artificial signal produced by varying the coefficient c - i.e. c0, c1, c2, c3 in the figure - is clearly visible. A corresponding representation is shown in Figure 5b, which shows the inset b from the Figure 3e. By means of the coefficients, the neural network is supplied with the temporal behaviour of the functions.

It is significant that the depicted moving average only requires two 32-bit RAM variables and an 8-bit ROM variable in order to generate the input variable in real time.

Neural network

In the input data of the neural network 40 in Figure 1, four main input variables are varied, i.e. four different moving averages (Matlab®) are calculated for the same:

- HRD(t)=f_ma(ABS(hr(t)-hr(t-1))))=(c*HRD(t-1)+ABS(hr(t)-hr(t-1))))/(c+1) - abs. fluctuation of heart rate - measures the absolute difference between the heart rate at time instant t, i.e. hr(t), and the average heart rate at time instant t-1, i.e. hr(t-1).
- MHR(t)=f_ma(MAX(0, hr(t)-min_hr))) - differentiable function of heart rate - measures the difference between the heart rate at time instant t, i.e. hr(t), and the minimum heart rate in the background parameters of the person, i.e.

min_hr.

- MAD(t)=f_ma(mad(t)) - inter-beat interval function - measures the mean absolute difference between successive RR-intervals.
- RESP(t)=f_ma(resp(t)) - respiratory rate function.

Moving average is calculated as f_ma(t)=(c*f_ma(t-1)+input(t))/(c+1), where input is the averaged data, which can be HRD, move_count, MHR, MAD, Resp or GRD, and c is a coefficient referring to a window length. In practice, c is an integer that defines how much the formerly calculated moving average f_ma(t-1) is weighted. If c is large, the new value input(t) has little effect on f_ma and the curve becomes very smooth. If c is small (e.g. 1 equals the average of two values) the curve is temporally more sensitive for changes, i.e. new values input(t) move the average quickly towards themselves.

In addition, three different moving averages are calculated for the GRD(t) input variable, GRD(t)=f_ma(MAX(0,hr(t)-min_hr)/MHRc(t)), where MHRc(t) is MHR(t) calculated with a chosen coefficient c, i.e. three MHR(t) variants are used here, thus producing three different GRD(t) values for a point in time t.

In the equation for GRD, the difference between the heart rate at time t, i.e. hr(t), and the minimum heart rate in the background parameters of the person, i.e. min_hr, is divided by MHRc(t). MHRc(t) is MHR(t) calculated with a selected coefficient c, i.e.

$$x(t)=MAX(0, hr(t)-min\_hr)))$$

$$MHR(t)=f\_ma(x(t)) =(x(t)+c1*f\_ma(x(t-1))/(1+c1),$$

$$MHR2(t)=f\_ma(x(t))=(x(t)+c2*f\_ma(x(t-1))/(1+c2),$$

$$MHR3(t)=f\_ma(x(t))=(x(t)+c3*f\_ma(x(t-1))/(1+c3),$$

$$MHR4(t)=f\_ma(x(t))=(x(t)+c4*f\_ma(x(t-1))/(1+c4),$$

where the coefficients c1, c2, c3, c4 refer to different time constants. When the MHRc(t) values are first calculated, the three different GRD(t) values can then be calculated as

$$GRD2(t)=f\_ma(MAX(0,hr(t)-min\_hr)/MHR2(t)),$$

$$GRD3(t)=f\_ma(MAX(0,hr(t)-min\_hr)/MHR3(t)),$$

$$GRD4(t)=f\_ma(MAX(0,hr(t)-min\_hr)/MHR4(t)),$$

where f_ma is calculated with coefficient c that is different to the coefficient c used in the calculation for MHRc. Coefficients c in the moving average for GRD can be, for example, 4, 8 and 12.

The foregoing describes input variables when heart-rate variability averages during sleep have not been calculated. If these are known, then the averaged input variables for HRD(t) and MAD(t), i.e. HRD2(t) and MAD2(t), respectively, are calculated as

- HRD2(t)=f_ma(abs(hr(t)-hr(t-1))/ave_hrd), where the difference between the heart rate at time instant t, i.e. hr(t), and the heart rate at time instant t-1, i.e. hr(t-1) is divided by the known average HRD value, i.e. ave_hrd, before the moving average is calculated.
- MAD2(t)=f_ma(mad(t)/ave_mad), where the mean absolute difference between successive RR-intervals is divided by the known average MAD value, i.e. ave_mad, before the moving average is calculated.

Moreover, two important main input variables for which separate temporal averages are not generated are move_count and detected time slept. Of these, the move_count is calculated as follows: the total absolute sum (act) for the three channels of the acceleration sensor is stored in 5s intervals and, on this basis, the moving average move_count(t)=(c*move_count(t-1)+(act>threshold))/(c+1) is calculated, where threshold is a value indicating significant movement, such as walking or moving hand rapidly. The detected time slept is stored to a sum register 52 which increases the cumulative sleep time when sleep is detected in block 51 (Figure 4).

In total, the neural network 40 thus has 21 input variables. The input data of the neural network 40 are scaled between 0-1.

The input data is fed into feed-forward neural network 40 divided into three subfunctions 41, 42, 43 as illustrated in the block diagram of Figure 7. The first neural network subfunction 41 (ffnet1) studies if the person is sleeping or not (sleep state 0). If sleep is detected in block 55 the calculation continues to the second neural network subfunction 42 (ffnet2), which detects the possible AWAKE state (sleep state 5). Furthermore, if awake state is not detected in block 56, the calculation continues to the third neural network subfunction 43 (ffnet3), which studies the DEEP / LIGHT / REM sleep (sleep state 2 - 4). If DEEP / LIGHT / REM sleep is detected the cumulative sleep time counter is updated and the updated value is forwarded to the next input. The feed-forward neural network analysis uses the following code in all three subfunctions:

```
 fxint ffnet(uint8 n0, uint8 n1, fxint input[], const int32 w[])
 {
  fxint finalbias = w[n0 * n1 + 2 * n1];
  for (uint8 l = 0; i < n1; i++) {
     fxint sum = w[i + n0 * n1 + n1];
     for (uint8 j = 0; j < n0; j++) {
     } sum += fx_mul(input[j], w[i * n0 + j]);
     finalbias += fx_mul(fx_log_sig(sum), w[i + n0 * n1]);
  }
 }return finalbias; // linear output
```

In the code above n0 is the number of inputs, n1 is the number of hidden neurons, vector input[] includes the input variables, and vector w[] includes the feed-forward neural network 40 parameters, which are different for all three subfunctions 41, 42, 43. The number of the neural network 40 weighting parameters, i.e. the size of vector w[], for the thee subfunctions 41, 42, 43 (ffnet1, ffnet2, ffnet3) is 22, 70, and 185, respectively. The number of hidden neurons in the thee subfunctions 41, 42, 43 (ffnet1, ffnet2, ffnet3) is 3, 3, and 8, respectively. The first subfunction 41 (ffnet1) utilizes only 5 selected input variables, while the other subfunctions (ffnet2 and ffnet3) utilize all 21 input variables. The feed-forward neural network parameters (number of inputs, number of hidden neurons and weightings) have been carefully selected, using empirical and physiological data described above and the Matlab software Neural Network Toolbox, in order to minimize the memory usage and calculation time while reaching an accurate result for the sleep detection. The output of the neural network analysis is a numerical value that is compared to a carefully selected threshold value in order to determine the sleep state.

Figure 6 shows the processing of the output of the neural network 40. The output of the neural network 40, i.e. the real-time values depicted in the middle bar of Figure 4, is simplified in block 66 and saved to host 60 in a simplified 8-bit format comprising the sleep state and the corresponding sleep state length of time. The raw output state values are also momentarily saved to 30 min loop buffer 62. The buffer 62 is read by auxiliary logic 64, which corrects the incoming signal in accordance with predetermined rules. The auxiliary logic 64 retrospectively corrects the state values to the host 60, and, thus, the final result obtained by means of a dynamic-delay calculation, depicted in the bottom bar of Figure 4, is obtained.

ROM/RAM requirements, comparison of the method

The input data (32 bit) according to the foregoing are

1: movecount

2-5 HRD

6-9 MHR

10-13 MAD

14-17: RESP

18-20: GRD

21: Sleep-time

Output (8 bit): Sleep state, the values of which are Blue: "deep sleep", Yellow: "light sleep"; Green: REM, Red: awake.

The method described here uses 21 input variables. [FITBIT] input variables contain several variables whose frequency must be calculated (VLF-, HF-, LF- power), of which *inter alia* the calculation of VLF power already requires the storage of a raw signal (0.015-0.4Hz) over a long period of time. [FBT] also uses a variable the rate of which must be calculated (resprate); however, the storage time required here is significantly shorter, respiratory rates generally being in the range >0.1Hz. Moreover, the size of the window significantly influences the calculation requirements of the Fourier transform in real time, as the so-called n^2 algorithm is used here.

[FITBIT] does not report the LKM parameters required by its model; however, the LKM of the input variables nevertheless relates to quite a large model for the large number input variables to be utilizable and in order to be able to distinguish between them within the model.

The method according to invention described here using 21 input variables for the feedforward-neural network model requires the storage of a total of 466 16-bit units in the ROM memory (0.932kb). The parameters of the model are optimized empirically e.g. by utilizing the Matlab neural network toolbox (or optimization toolbox).

The dynamic delay is realized with two average windows, of which the larger window (25min) defines the number of sleep states to be stored (the smaller window is 5min). As it is not worth calculating sleep states with a resolution greater than 1min, a storage (51 bytes) of 51 8-bit values in a loop buffer is sufficient for this purpose. Other dynamic-delay logics (inter alia "feedback synthesis") require the maintenance of three uint16 time indexes in real time.

In addition to the foregoing, cumulative values such as times slept in different sleep states are calculated and the values of the night-time heart-rate variability variables ave_hrd, ave_mad are updated in the moving average windows for the provision of feedback statements.

```
#define SLEEP_STAGE_INPUT_SIZE 21
#define SLEEP_STAGE_DIV 12 // calculation update 5s, DIV=12 => resolution for sleep calculation
1min
#define SLEEP_STAGE_N (612/SLEEP_STAGE_DIV)
typedef struct {
 int8 lag_state[2];
 uint8 acw, i, ffnet_index, state, n_act;
 uint16 ave_n, state_sum[2][5], sleep_time[2][4], t1, t2, lag_len, lag_t, an,fr1,fr2,fr3;
 uint32 ave_sleep[3], lst, lst4h, act;
 fxint ave_sleep_hrd[2], ave_sleep_mad[2];
 fxint input[2][SLEEP_STAGE_INPUT_SIZE]; // Sleep Stage Detection input- NN Input layer
 uint8 sleep[SLEEP_STAGE_N]; // loop buffer for sleep states, resolution SLEEP_STAGE_DIV
} iete_sleep_stage_variables;
```

In all, the part required for sleep detection uses a dynamic memory in accordance with the structure described above (2*8+5*8+27*16+6*32+4*32+42*32)/8=269 bytes=0.269kb.

**Claims**

1. Computer implemented method for detecting a sleep state by means of heart-rate and movement data using a device with a CPU (32), ROM and RAM memory (31.1, 31.2) and software, in which method heart rate data including inter-beat interval data is measured by a first sensor and movement data is measured by a second sensor, a correlation function is used for detecting a sleep state having input variables, which includes first variable derived from heart rate data in terms of beats per minute, such as heart rate deviation (HRD), and second variable derived from movement data, **characterized in that** the correlation function has further inputs

   - at least one variable as third variable derived from inter-beat interval data in terms of milliseconds, such as mean absolute difference between successive RR-intervals (MAD),
   - a fourth variable depicting cumulative sleep time, and
   - said variable derived from heart rate data includes at least one of the following: heart rate deviation (HRD) and heart rate difference (from minimum HR) (MHR),

variables derived from the heart-rate and/or inter-beat interval data (such as HRD, MHR, MAD) are further modified by 0-4 artificial average functions having different weightings, and

the total number of inputs for the correlation function is 7 - 28, preferably 17 - 23.

2. Method according to claim 1, **characterized in that** neural network software is used for detecting a sleep state and the input data are movement data (move_count) and data derived from heart-rate data and data derived from inter-beat interval data and respiration data (RESP), wherein

the time changing variables used as input data for the neural network software are

- move count
- heart rate deviation (HRD)
- heart rate difference (from minimum HR) (MHR)
- mean absolute difference between successive RR-intervals (MAD)
- respiration (Resp)
- gradient of the heart rate difference (GRD),
- cumulative sleep time, and

at least a portion of the variables (HRD, MHR, MAD, Resp, GRD) derived from the heart-rate and/or inter-beat interval data are further modified by 2-4 artificial average functions, which have the form $f\_ma(t) = (c*f\_ma(t-1+input(t))/(c+1)$, where c is 20-80 with a sample window of 5s and input is the input data to be averaged.

3. Method according to claim 1, **characterized in that** respiration (Resp) data modified by 0-4 artificial average functions having different weightings is also used as input data for the correlation function.

4. Method according to claim 1 or 3, **characterized in that** gradient data of the heart rate difference (GRD) modified by 0-4 artificial average functions having different weightings is also used as input data for the correlation function.

5. Method according to claims 1 or 3 - 4, **characterized in that** the average function has the form $f\_ma(t) = (c*f\_ma(t-1+input(t))/(c+1)$, where c is typically 20 - 80 with a sample window of 5 s and input is the input data to be averaged.

6. Method according to claims 1 or 3 - 5, **characterized in that** the correlation function is a neural network, preferably a feed-forward neural network.

7. Method according to claims 1-6, **characterized in that** the correlation function includes at least two subfunctions where the first subfunction detects only sleep states "sleep" & "non-sleep", and where the rest of the subfunctions handle said sleep states consisting several different states.

8. Method according to claims 1-7, **characterized in that** the heart-rate data and night-time inter-beat interval data are generated with a PPG device.

9. Method according to claims 1-8, **characterized in that** in addition to the real-time detection of the method, a correction is calculated during a dynamic delay so that a time window of a selected length, e.g. a five-minute time window, is applied to the incoming signal, whereupon an auxiliary logic corrects the incoming signal in accordance with predetermined rules so that the episodes constituting the bulk of one sleep state are first sought in the incoming signals, that part is selected in accordance with a main rule, e.g. 80% of the episodes, and, apart from previously defined exceptions, the whole episode is modified in accordance with the bulk of the sleep state.

10. Method in accordance with claim 9, **characterized in that,** as a special rule, AWAKE states are not modified in accordance with the bulk of the state.

11. Method according to claims 1-10, **characterized in that**

the calculation of the night-time averages of the heart-rate variability variables occurs in the method in such a way that two neural models are implemented as follows:

during the measurement the first night, a less accurate neural model is used for the detection of a sleep state and an average of the heart-rate variability variables is stored in real time, e.g. the first 4h of the night are a sufficient sample for the variables to stabilize, after which it is possible to switch to using a neural model that utilizes these averages.

**12.** Method according to claim 11, **characterized in that** the values of the heart-rate variability variables are distributed in the calculation by means of the average night-time heart-rate variability.

**13.** Method according to claim 11 or 12, **characterized in that** the method includes two different neural models for the detection, of which the first (the less accurate) is used when the night-time background parameters have not yet been calculated and the subsequent method is used when the background parameters have been calculated.

**14.** An apparatus for detecting a sleep state of a user, the apparatus including a software-operating system, comprising a CPU (32), RAM and ROM memory (31.1, 31.2), heart-rate sensor (12), accelerometer (70), input unit (31) for receiving heart-rate data and movement data, an output unit (34) and a software including a correlation function, said software being arranged to monitor sleep of the user using data provided by the heart-rate sensor (12) and accelerometer (70) and to calculate the sleep state using the correlation function, said RAM memory (31.1) including

- a result register for storing sleep states during selected time period,
- a sum register for storing cumulative sleep time,
**characterized in that** the input data for the correlation function are
- movement data (move count),
- at least one variable derived from heart-rate data, such as heart rate deviation (HRD) and heart rate difference (from minimum HR) (MHR),
- at least one variable derived from inter-beat interval data, such as mean absolute difference between successive RR-intervals (MAD),
- cumulative sleep time, and
variables derived from the heart-rate and/or inter-beat interval data (such as HRD, MHR, MAD) are further modified by 0-4 artificial average functions having different weightings, and
the total number of inputs for the correlation function is 7 - 28, preferably 17 - 23, and software further being arranged
- to sum the detected sleep time and store it to said sum register,
- to set input data to the said correlation function, and
- to calculate the resulting sleep state.

**15.** Apparatus according to claim 14, **characterized in that** the apparatus includes a PPG wrist device as its only source of heart-rate data.

**16.** Apparatus according to claim 14 or 15, **characterized in that** the apparatus includes an ECG device as its only source of heart-rate data.

**17.** Apparatus according to claims 14 - 16, **characterized in that** the apparatus comprises a buffer memory to temporarily store sleep state data and a register to store auxiliary logic that corrects the last periods of sleep states in accordance with predetermined rules.

Fig. 1

Fig.2

EP 3 753 482 A1

Fig. 3a

Fig. 3b

Fig. 3c

Fig. 3d

Fig. 3e

Fig. 3f

Fig. 3g

s17₂0180219₁53836₈0501.mat. Blue deep sleep 1.1h, Yellow light sleep 5.6h, green REM 1.7h, red awake 0.8h, total 9.3h

G data84: f1=64.40%1.4h/5.6h/1.8h/0.8h, total 9.7h

data84: f1=68.75%1.1h/5.4h/2.1h/0.6h, total 9.1h

Time (min), 2018-02-19 15:38:36

Fig. 4

EP 3 753 482 A1

Fig. 5a

Fig. 5b

Fig. 6

Fig. 7

sleep_n=0

sleep_n=5

sleep_n=2,3,4

DEEP /
LIGHT /
REM

Europäisches
Patentamt

European
Patent Office

Office européen
des brevets

# EUROPEAN SEARCH REPORT

Application Number

EP 20 17 5446

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | US 2017/215808 A1 (SHIMOL DAVID BEN [US] ET AL) 3 August 2017 (2017-08-03) * paragraphs [0041] - [0092]; figures 3-9 * | 1-17 | INV. A61B5/0205 A61B5/024 A61B5/08 A61B5/11 A61B5/00 |
| Y | CN 108 992 040 A (SHENZHEN ZHIXIN DATA SERVICE CO LTD) 14 December 2018 (2018-12-14) * the whole document * | 1-17 | ADD. A61B5/04 |
| X | US 2018/064388 A1 (HENEGHAN CONOR JOSEPH [US] ET AL) 8 March 2018 (2018-03-08) * paragraphs [0016], [0023], [0043], [0047], [0050] * * paragraphs [0080] - [0085] * * paragraphs [0116] - [0117] * * paragraphs [0140] - [0179] * * page f; figures 1-12 * | 1,14 2-13, 15-17 | |
| A | US 2016/007934 A1 (ARNOLD JACOB ANTONY [US] ET AL) 14 January 2016 (2016-01-14) * paragraphs [0110] - [0130] * | 1-17 | TECHNICAL FIELDS SEARCHED (IPC) A61B |
| A | US 2019/053754 A1 (GOWDA SURAJ [US] ET AL) 21 February 2019 (2019-02-21) * paragraphs [0057] - [0058] * | 1,2,14 | |
| A | WO 2018/221750 A1 (UNIV KEIO [JP]) 6 December 2018 (2018-12-06) * paragraphs [0024] - [0056] * | 1-17 | |

The present search report has been drawn up for all claims

| Place of search | Date of completion of the search | Examiner |
|---|---|---|
| Berlin | 12 November 2020 | Dydenko, Igor |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 20 17 5446

This annex lists the patent family members relating to the patent documents cited in the above-mentioned European search report.
The members are as contained in the European Patent Office EDP file on
The European Patent Office is in no way liable for these particulars which are merely given for the purpose of information.

12-11-2020

| Patent document cited in search report | | Publication date | Patent family member(s) | | Publication date |
|---|---|---|---|---|---|
| US 2017215808 | A1 | 03-08-2017 | NONE | | |
| CN 108992040 | A | 14-12-2018 | NONE | | |
| US 2018064388 | A1 | 08-03-2018 | CA | 3035795 A1 | 15-03-2018 |
| | | | CN | 109843163 A | 04-06-2019 |
| | | | EP | 3509482 A2 | 17-07-2019 |
| | | | US | 2018064388 A1 | 08-03-2018 |
| | | | WO | 2018048951 A2 | 15-03-2018 |
| US 2016007934 | A1 | 14-01-2016 | CN | 105446480 A | 30-03-2016 |
| | | | CN | 110083236 A | 02-08-2019 |
| | | | CN | 110083237 A | 02-08-2019 |
| | | | US | 2016007934 A1 | 14-01-2016 |
| | | | US | 2016022175 A1 | 28-01-2016 |
| | | | US | 2016022201 A1 | 28-01-2016 |
| | | | US | 2016022203 A1 | 28-01-2016 |
| | | | US | 2019038185 A1 | 07-02-2019 |
| US 2019053754 | A1 | 21-02-2019 | CN | 111246798 A | 05-06-2020 |
| | | | EP | 3668396 A1 | 24-06-2020 |
| | | | US | 2019053754 A1 | 21-02-2019 |
| | | | US | 2019282159 A1 | 19-09-2019 |
| | | | WO | 2019036661 A1 | 21-02-2019 |
| WO 2018221750 | A1 | 06-12-2018 | EP | 3632322 A1 | 08-04-2020 |
| | | | JP | 6727432 B2 | 22-07-2020 |
| | | | JP | WO2018221750 A1 | 25-06-2020 |
| | | | WO | 2018221750 A1 | 06-12-2018 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- Estimation of sleep stages in a healthy adult population from optical plethysmography and accelerometer signals. *Physiol Meas,* 31 October 2017, vol. 38 (11), 1968-1979

- **BEATTIE Z. et al.** *Fitbit Research, San Francisco*
- **BEATTIE.** *Estimation of sleep stages in a healthy adult population from optical plethysmography and accelerometer signals,* 2017